Europäisches Patentamt

European Patent Office

Office européen des brevets

(11) Numéro de publication: **0 191 253**
A2

## (12) DEMANDE DE BREVET EUROPEEN

(21) Numéro de dépôt: 85402132.6

(22) Date de dépôt: 06.11.85

(51) Int. Cl.⁴: **A 61 K 31/42**

(30) Priorité: 07.11.84 FR 8417051

(43) Date de publication de la demande: **20.08.86 Bulletin 86/34**

(84) Etats contractants désignés: **AT BE CH DE FR GB IT LI LU NL SE**

(71) Demandeur: **Albert ROLLAND S.A. Société dite, 49, Rue St-André-des-Arts, F-75006 Paris (FR)**

(72) Inventeur: **Rolland, Anne, 49, Rue Lamarck, F-75018 Paris (FR)**

(74) Mandataire: **Burtin, Jean-François, 5 Bis, rue Parmentier, F-92200 Neuilly S/Seine (FR)**

(54) **Compositions pharmaceutiques à base de 3-phényl-4-diéthylamino-éthyl-5-imino-1,2,4-oxadiazoline en tant qu'agents vaso-constricteur.**

(57) L'invention concerne une nouvelle application thérapeutique du 3-phényl 4-diéthylamino éthyl 5-imino oxadiazole ou d'un de ses sels.

Elle concerne l'utilisation sous forme de compositions pharmaceutiques renfermant de 30 à 300 mg de principe actif par prise unitaire pour le traitement des troubles de la circulation veineuse.

Ces compositions pharmaceutiques sont utilisées dans l'industrie pharmaceutique comme médicament veinotonique.

ACTORUM AG

Nouvelle utilisation thérapeutique du 3-phenyl 4-diethyl amino-ethyl 5-imino oxadiazole (1, 2 , 4) et de ses sels ainsi que les compositions pharmaceutiques préparées à cet effet.

-------------

Le 3-phenyl 4-diethylaminoethyl 5-imino oxadiazole (1,2,4) est connu sous la dénomination commune d'Imolamine.

Ce composé a déjà trouvé depuis de nombreuses années un usage en thérapeutique humaine pour le traitement de l'angine de poitrine. Il a été protégé en tant que substance par le brevet français N° 1.351.391.

Or, il a été trouvé maintenant que l'Imolamine et sels d'addition avec un acide minéral ou organique provoque une vasoconstriction veineuse. Ces propriétés ont été retrouvées expérimentalement chez l'animal et chez l'homme.

L'Imolamine a montré des propriétés veinotoniques chez l'animal aussi bien in vitro qu'in vivo :

<u>In vitro</u> :

L'Imolamine provoque une constriction des veines isolées variées (saphène de chien, veine-porte de rat) à des concentrations allant de 100 µg à 1 mg/ml de bain.
Aucune activité vasoconstrictrice n'est constatée sur l'artère isolée en particulier sur l'aorte de rat à des concentrations 5 à 10 fois supérieures.

<u>In vivo</u> :

Chez le cobaye, l'Imolamine protège l'animal contre l'apparition de pétéchies provoquée par dépression au niveau de la peau. Les doses actives varient entre 5 et 20 mg/kg I.P.

Chez l'homme volontaire sain, les propriétés veinoconstrictrices de l'Imolamine ont été mises en évidence par deux méthodes :

. Une première méthode consiste à photographier les variations des diamètres veineux. Un appareil photographique équipé d'un flash et de lentilles adaptées est dirigé sur une veine dorsale de main, le bras et la main étant dans une position bien définie.

Un brassard, au niveau du bras, est gonflé à une pression de 44 mmHg, et une photographie est prise après 1mn; le brassard est ensuite dégonflé et 30sec. après une deuxième photographie est prise. La différence entre les 2 diamètres veineux ainsi mesurés est considérée comme représentant la variation du diamètre de la veine.

. Une deuxième méthode utilise un microscope qui mesure les changements de diamètre veineux. Cette méthode a été décrite par Nachev, Collier et Robinson (1971) et modifiée par Aellig (1975).

La première méthode a été utilisée chez 8 sujets volontaires sains, la deuxième méthode chez 6 de ces 8 sujets.

L'étude réalisée en double aveugle en comparaison avec un placebo a consisté à mesurer les modifications des diamètres veineux après perfusion de 200 mg d'Imolamine ou de placebo aux temps 0, 5 mn, 15 mn, 30 mn, 45 mn, 1 h, 1 h 30, 2 h, 2 h 30, 3 h, 4 h, 6 h.

La perfusion d'Imolamine entraîne une veinoconstriction tout de suite après la perfusion et persistant près de 2 heures d'une manière variable selon les sujets. La veinoconstriction maximale est atteinte en moyenne 45mn après la perfusion. Ces résultats sont statistiquement différents de ceux obtenus avec le placebo ($p < 0,01$).

En tant qu'agent veinoconstricteur, l'Imolamine ou un de ses sels d'addition est présentée sous forme de compositions pharmaceutiques. Celles-ci comportent un ou plusieurs diluants, un agent d'adhésion, un agent tensio actif, un agent liant, un agent solubilisant et/ou un agent aromatisant.

0191253

- 4 -

Les compositions pharmaceutiques pour cette nouvelle utilisation thérapeutique sont celles qui conviennent pour l'administration par voie parentérale, buccale, rectale, sublinguale ou percutanée.

On pourra citer en particulier les comprimés nus ou enrobés, les gélules, les gouttes, les solutions buvables ou injectables, les suppositoires, les comprimés sub-linguaux, les préparations dermatologiques, les préparations dans un solvant polaire pénétrant.

La posologie moyenne unitaire s'échelonne de 30 à 300 mg et la posologie quotidienne s'échelonne de 60 à 600 mg.

Les compositions pharmaceutiques pour cette nouvelle indication thérapeutique sont destinées au traitement des troubles de la circulation veineuse comme, par exemple, les varices, les plaies ulcéreuses, le syndrome des jambes lourdes, les hémorroïdes ou les phénomènes congestifs du syndrome prémenstruel, l'hypotension orthostatique, les lymphoedèmes.

L'exemple suivant met en évidence les propriétés veinoconstrictrices du 3-phenyl 4-diethylamine ethyl 5-imino oxadiazole (1, 3, 4) ou de ses sels. Il est fourni à titre simplement illustratif.

EXEMPLE

L'objectif et le but de l'expérience menée sur huit volontaires sains a été de mettre en évidence des propriétés veinoconstrictives après perfusion de l'Imolamine à une dose de 200 mg par voie intraveineuse. Cette étude a été faite en double aveugle vis-à-vis d'un placebo sur 8 sujets masculins tirés au sort et répartis au hasard en deux groupes. L'Imolamine a été mise en solution dans 160 ml de serum physiologique et perfusée à la vitesse de 300 ml/heure pendant 20 minutes.

La pression artérielle et le pouls ont été enregistrés pendant la perfusion et jusqu'à 6 heures après l'arrêt de la perfusion.

../..

- 5 -

La détermination du diamètre de la veine dorsale de la main est effectuée aux temps 0 (pre dose immédiate), 5 mn, 15 mn, 30 mn, 45 mn, 1 h, 1 h 30, 2 h, 2 h 30, 3 h, 4 h et 6 h après l'achèvement de la perfusion.

L'Imolamine ne manifeste sur ces tests aucun effet vasodilatateur qui soit significatif. Les valeurs trouvées montrent que les variations moyennes de pourcentage de vélocité du sang dans l'artère radiale sont très similaires pour l'Imolamine et le placebo.

Par contre, l'Imolamine amène une plus grande vélocité sanguine, supérieure à celle amenée par le placebo. Néanmoins, la seule différence importante se manifeste au maximum de l'augmentation pour chaque sujet. Cette différence est statistiquement significative à $p$ 0.05. L'apparition de ce maximum varie selon les sujets. Il s'échelonne entre 5 mn et 6 h après la perfusion avec une valeur moyenne de 2 heures. C'est pourquoi on peut considérer que ce petit effet n'apparait qu'avec un certain retard. Etant donné que la demi-vie de l'Imolamine est d'environ 1 heure 30 minutes, il est vraisemblable que cet effet apparait lorsque moins de la moitié de la substance ne disparait de la circulation sanguine.

Le test du diamètre de la veine dorsale de la main met en évidence l'effet veino-constricteur de l'Imolamine. La méthode microscopique utilisée sur les 8 sujets montre qu'immédiatement après la fin de la perfusion il se manifeste une veino-constriction importante, statistiquement significative en comparaison avec l'effet du placebo. Cet effet ne se maintient pas au maximum mais il persiste d'une manière significative pendant plus de deux heures. Le maximum se maintient pendant environ 45 minutes.

Une autre détermination a été effectuée à l'aide d'une camera sur six des huit sujets seulement et confirme les résultats obtenus pendant la période de veinoconstriction maximale.

En conséquence, les effets veinoconstricteurs de l'Imolamine se manifestent principalement tout de suite après la perfusion mais persistent pendant près de deux heures d'une manière variable selon les sujets.

../..

0191253

- 6 -

La veinoconstriction maximale est atteinte en moyenne 45 minutes après la perfusion et son degré de significativité par rapport au placebo est $\leqslant 0.01$.

Les tableaux I et II rassemblent les valeurs des variations de diamètre obtenues respectivement avec le placebo et avec l'Imolamine (Annexes 1, 2 et 3).

% DE MODIFICATION DU DIAMETRE DE LA VEINE DORSALE DE LA MAIN (METHODE D'OBSERVATION AU MICROSCOPE) DU DEBUT DE LA PERFUSION A 20 MINUTES APRES LA PERFUSION DU PLACEBO CHEZ HUIT SUJETS SAINS ADULTES.

| Sujet Nr. Temps | 1 | 2 | 3 | 4 | 5 | 6 | 7 | 8 | Moyenne | Standard Deviation |
|---|---|---|---|---|---|---|---|---|---|---|
| 0 min | -66.67 | -25.00 | 23.08 | -16.67 | 28.57 | NR | -38.89 | 25.00 | -16.68 | 33.88 |
| 5 min | -26.67 | -35.00 | 30.77 | 5.56 | 100.00 | -100.00 | -22.22 | 0.00 | -5.95 | 57.48 |
| 15 min | -20.00 | -25.00 | 61.54 | -44.44 | 271.43 | -62.50 | -16.67 | 66.67 | 28.88 | 108.51 |
| 30 min | -6.67 | -15.00 | -15.38 | -27.78 | 185.71 | -6.25 | 22.22 | 16.67 | 19.19 | 69.31 |
| 45 min | 33.33 | -5.00 | 92.31 | 50.00 | NR | NR | -16.67 | -8.33 | 24.27 | 42.35 |
| 1 hr | -20.00 | -60.00 | 38.46 | -11.11 | 14.29 | -68.75 | 22.22 | -8.33 | -11.65 | 37.82 |
| 1½ hr | 6.67 | -75.00 | 30.77 | 5.56 | 228.57 | -68.75 | -38.89 | 50.00 | 17.37 | 96.52 |
| 2 hr | 26.67 | -70.00 | 76.92 | 16.67 | 142.86 | 31.25 | -5.56 | 25.00 | 30.48 | 61.43 |
| 2½ hr | -33.33 | -5.00 | 61.54 | -27.78 | 100.00 | -87.50 | -27.78 | -25.00 | -5.61 | 59.16 |
| 3 hr | -26.67 | -30.00 | -53.85 | 33.33 | 114.29 | - | -38.89 | -25.00 | -3.83 | 58.76 |
| 4 hr | 13.33 | -35.00 | NR | -66.67 | -42.86 | -43.75 | -55.56 | 50.00 | -25.79 | 41.89 |
| 6 hr | -40.00 | -25.00 | NR | 5.56 | 85.71 | -62.50 | -16.67 | 0.00 | -7.56 | 47.23 |

NR = PAS DE RESULTAT DISPONIBLE

% DE MODIFICATION DU DIAMETRE DE LA VEINE DORSALE DE LA MAIN (METHODE D'OBSERVATION
AU MICROSCOPE) DU DEBUT DE LA PERFUSION A 20 MINUTES APRES LA PERFUSION DE L'IMOLAMINE
CHEZ HUIT SUJETS SAINS ADULTES.

| Temps \ Sujet Nr. | 1 | 2 | 3 | 4 | 5 | 6 | 7 | 8 | Moyenne | Standard Deviation |
|---|---|---|---|---|---|---|---|---|---|---|
| 0 min | -68.18 | -53.85 | -21.43 | -47.62 | NR. | -88.89 | -77.78 | -30.77 | -55.50 | 24.53 |
| 5 min | -68.18 | -100.00 | -50.00 | NR. | -100.00 | -55.56 | -16.67 | -61.54 | -64.56 | 29.21 |
| 15 min | -40.91 | -23.08 | 0.00 | 19.05 | -80.00 | -11.11 | 0.00 | -53.85 | -23.74 | 32.70 |
| 30 min | -95.45 | 0.00 | 0.00 | -19.05 | 13.33 | 133.33 | 11.11 | -100.00 | -7.09 | 72.85 |
| 45 min | -63.64 | 15.38 | -7.14 | -61.90 | 93.33 | -44.44 | -22.22 | 15.38 | -9.41 | 51.93 |
| 1 hr | -81.82 | -7.69 | 35.71 | -23.81 | -80.00 | -33.33 | 16.67 | -69.23 | -30.44 | 44.35 |
| 1½ hr | -68.18 | -38.46 | 78.57 | -42.86 | 0.00 | -55.56 | -11.11 | -61.54 | -24.89 | 48.07 |
| 2 hr | -36.36 | 15.38 | -21.43 | 4.76 | 26.67 | 22.22 | -50.00 | -100.00 | -17.35 | 43.65 |
| 2½ hr | -36.36 | 15.38 | 14.29 | 9.52 | 20.00 | -55.56 | -38.89 | -100.00 | -21.45 | 43.38 |
| 3 hr | 9.09 | -7.69 | 7.14 | -28.57 | 33.33 | -66.67 | -22.22 | -30.77 | -13.30 | 30.69 |
| 4 hr | -13.64 | -7.69 | -28.57 | -38.10 | -100.00 | -44.44 | -22.22 | 0.00 | -31.83 | 31.34 |
| 6 hr | -81.82 | -7.69 | -7.14 | -14.29 | 20.00 | 33.33 | -27.78 | -38.46 | -15.48 | 35.56 |

NR. = PAS DE RESULTAT DISPONIBLE

8

0191253

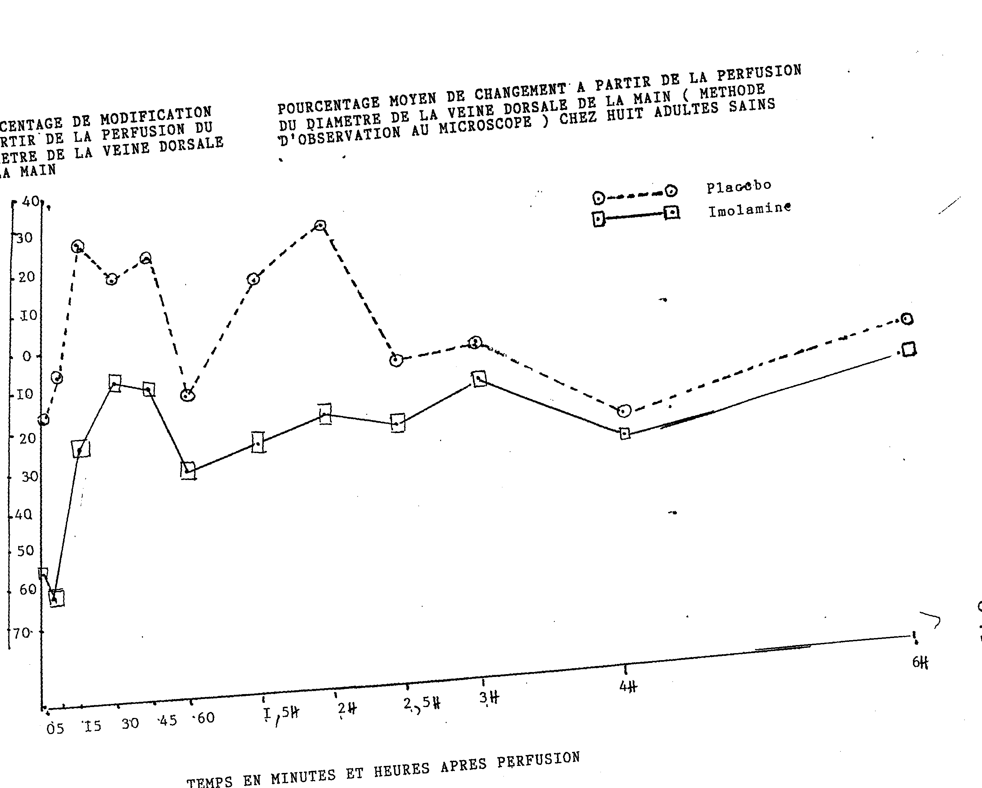

REVENDICATIONS

L'invention a pour objet :

1/ L'emploi du 3-phényl 4-diethylamino éthyl 5-imino oxadiazole (1,2,4) ou de ses sels d'addition avec un acide minéral ou organique en tant qu'agent vasoconstricteur veineux.

2/ Les compositions pharmaceutiques renfermant du 3-phényl 4-diéthylamino éthyl 5-imino oxadiazole (1,2,4) ou un de ses sels d'addition spécialement présentées pour l'usage défini à la revendication 1/.

3/ Les compositions pharmaceutiques selon la revendication 2/ renfermant de 30 à 300 mg de principe actif par prise unitaire en association ou en mélange avec un véhicule ou un excipient inerte non-toxique pharmaceutiquement acceptable.

4/ Les compositions pharmaceutiques selon l'une des revendications 2/ ou 3/ présentées sous l'une des formes appropriées pour l'administration par voie parentérale, buccale, rectale, perlinguale ou cutanée.

5/ Un procédé d'obtention des formes pharmaceutiques à action vasoconstrictrice selon l'une des revendications 2/ à 4/ caractérisé en ce que l'on mélange ou ajoute du 3-phényl 4-diéthylaminoéthyl 5-imino oxadiazole (1,2,4) ou un de ses sels d'addition avec un acide minéral ou organique à un excipient ou à un véhicule inerte non-toxique, pharmaceutiquement-compatible.

POURCENTAGE DE MODIFICATION A PARTIR DE LA PERFUSION DU DIAMETRE DE LA VEINE DORSALE DE LA MAIN

POURCENTAGE MOYEN DE CHANGEMENT A PARTIR DE LA PERFUSION DU DIAMETRE DE LA VEINE DORSALE DE LA MAIN ( METHODE D'OBSERVATION AU MICROSCOPE ) CHEZ HUIT ADULTES SAINS

Placebo
Imolamine

TEMPS EN MINUTES ET HEURES APRES PERFUSION